(19) Europäisches Patentamt
European Patent Office
Office européen des brevets

(11) **EP 1 711 776 B1**

(12) **FASCICULE DE BREVET EUROPEEN**

(45) Date de publication et mention de la délivrance du brevet:
**27.04.2016 Bulletin 2016/17**

(51) Int Cl.:
***G01B 9/02*** *(2006.01)*  ***A61B 3/12*** *(2006.01)*

(21) Numéro de dépôt: **05717464.1**

(86) Numéro de dépôt international:
**PCT/FR2005/000135**

(22) Date de dépôt: **21.01.2005**

(87) Numéro de publication internationale:
**WO 2005/080912 (01.09.2005 Gazette 2005/35)**

(54) **DISPOSITIF ET PROCEDE POUR MESURER LE CONTRASTE DES FRANGES DANS UN INTERFEROMETRE DE MICHELSON, ET SYSTEME D'EXAMEN DE L'OEIL INCLUANT UN TEL DISPOSITIF**

EINRICHTUNG UND VERFAHREN ZUR MESSUNG DES KONTRASTS DER RÄNDER IN EINEM MICHELSON-INTERFEROMETER UND SYSTEM ZUM UNTERSUCHEN DES AUGES MIT EINER SOLCHEN EINRICHTUNG

DEVICE AND METHOD FOR MEASURING THE CONTRAST OF THE FRINGES IN A MICHELSON INTERFEROMETER AND SYSTEM FOR EXAMINATION OF THE EYE COMPRISING SUCH A DEVICE

(84) Etats contractants désignés:
**AT BE BG CH CY CZ DE DK EE ES FI FR GB GR HU IE IS IT LI LT LU MC NL PL PT RO SE SI SK TR**

(30) Priorité: **22.01.2004 FR 0400580**

(43) Date de publication de la demande:
**18.10.2006 Bulletin 2006/42**

(73) Titulaires:
• **CENTRE NATIONAL DE LA RECHERCHE SCIENTIFIQUE -CNRS-**
**75794 Paris Cedex 16 (FR)**
• **Observatoire de Paris**
**75014 Paris (FR)**
• **Mauna Kea Technologies**
**75010 Paris (FR)**

(72) Inventeurs:
• **LACOMBE, François**
**F-92370 Chaville (FR)**
• **LAFAILLE, David**
**F-76600 LE HAVRE (FR)**

(74) Mandataire: **Pontet Allano & Associes**
**Parc Les Algorithmes, Bâtiment Platon**
**CS 70003 Saint-Aubin**
**91192 Gif-sur-Yvette Cedex (FR)**

(56) Documents cités:
FR-A- 2 828 396     US-A- 3 849 001
US-A- 4 541 697     US-A- 5 239 411
US-A- 5 877 856     US-A- 5 973 784

• **C. K. HITZENBERGER ET AL.: "DIFFERENTIAL PHASE CONTRAST IN OPTICAL COHERENT TOMOGRAPHY" OPTICS LETTERS, vol. 24, no. 9, 1 mai 1999 (1999-05-01), pages 622-624, XP001183361**

**Description**

La présente invention concerne un dispositif pour mesurer le contraste des franges dans un interféromètre de Michelson opérant en plein champ. Elle vise également un procédé mis en oeuvre dans ce dispositif, ainsi qu'un système d'examen de l'oeil incluant un tel dispositif.

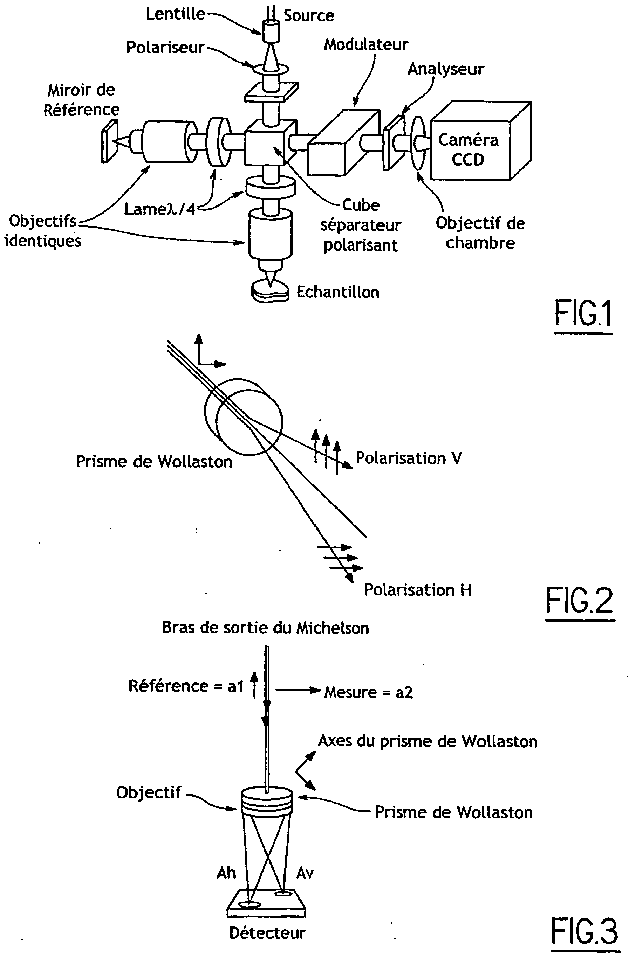

L'interférométrie de Michelson plein champ est une technique interférométrique dérivée du montage de Michelson classique qui permet entre autres de déduire les profils de surface et de réflectance d'un échantillon à partir des interférences réalisées entre la lumière réfléchie par l'échantillon et celle en provenance d'un bras de référence. Lorsqu'elle est mise en oeuvre avec une source de lumière temporellement faiblement cohérente, cette technique présente dans les milieux transparents et faiblement diffusants une capacité tomographique. Il s'agit alors de tomographie optique cohérente (OCT) plein champ. La figure 1 illustre ainsi la structure d'un système de tomographie OCT par interférométrie plein champ représentatif de l'art antérieur.

Pour l'étude de milieux éventuellement biréfringents, d'une part, mais également dans le souci de collecter le maximum de lumière en retour de l'échantillon, l'OCT plein champ a souvent été utilisée en lumière polarisée. Dans ce cas, les deux bras de l'interféromètre de Michelson fonctionnent avec des polarisations perpendiculaires. Les interférences ne sont réalisées sur le bras de sortie qu'après projection sur un axe commun par l'emploi d'un analyseur à 45 degrés.

La modulation interférométrique, jusqu'à présent nécessaire à la détection d'interférences de faible amplitude, est réalisée soit en déplaçant le miroir mobile du bras de référence, soit en modulant le chemin optique d'une des polarisations sur le bras de sortie de l'interféromètre, par exemple, par l'emploi avant l'analyse à 45 degrés, d'un modulateur photo-élastique. Les mesures d'amplitude d'interférogrammes dans des conditions médiocres de rapport signal/bruit amènent souvent à considérer des techniques de modulation de la différence de marche interférométrique couplées à des techniques de détection synchrone.

L'état de phase des interférogrammes obtenus va être temporellement modifié de manière à pouvoir éliminer a composante continue éventuellement variable du signal mesuré, qui résulte de la combinaison incohérente des signaux de sortie, due soit à des réflexions parasites, soit à des réflexions avec des différences de marche supérieures à la longueur de cohérence du rayonnement utilisé.

Quand la stabilité mécanique, donc interférométrique, de l'ensemble du système impose des fréquences de modulation élevées, il arrive que la chaîne de détection, le plus souvent un capteur CCD dans le cas du plein champ, ne puisse opérer convenablement. En général, celle-ci devient le facteur limitant de la fréquence de modulation/détection donc de la capacité du système à observer des franges.

Lors de l'analyse d'un interférogramme de Michelson, au moins trois mesures de l'interférogramme à des états de phase différents sont nécessaires pour déduire sans ambiguïté l'amplitude et la phase de l'interférogramme. Pour des raisons pratiques, on opte le plus souvent pour quatre mesures indépendantes obtenues pour des différences de marche séparées d'un quart de longueur d'onde. C'est donc quatre mesures qu'il est nécessaire de réaliser dans le temps caractéristique où le système d'interférences peut être considéré comme stable. Cette exigence rend d'autant plus délicate la mise en oeuvre d'une détection synchrone.

Quand la stabilité temporelle de l'interférogramme fait défaut, ou que la chaîne de détection ne peut atteindre les fréquences requises, la mesure devient difficile, voire impossible.

En sortie d'un interféromètre de Michelson, on utilise habituellement un polariseur à 45 degrés des directions de polarisation incidentes, sur la direction duquel les champs issus des deux bras de l'interféromètre sont projetés. Apparaissent alors les interférences. Lors de cette projection, la moitié de l'énergie entrante est perdue, ce qui contribue à dégrader les performances de l'interféromètre.

Le but de la présente invention est de remédier à cet inconvénient en proposant un dispositif pour mesurer le contraste des franges dans un interféromètre de Michelson, permettant une obtention simultanée d'interférogrammes dans différents états de phase, ce qui donne accès à une mesure de leur amplitude et même de leur phase en dépit d'éventuelles instabilités. Cette invention s'applique au cas d'interférogrammes plein champ en lumière polarisée.

Cet objectif est atteint avec un dispositif de mesure de contraste comprenant des moyens pour dévier deux polarisations perpendiculaires entrantes dans deux directions émergeantes différentes, ces moyens de déviation étant disposés au sein de l'interféromètre en substitution du polariseur unique.

La lumière perdue est la projection des champs sur une direction perpendiculaire à la direction du polariseur. Or sur cette direction, on pourrait observer aussi des interférences. Remplacer le polariseur unique par un prisme de Wollaston permet d'utiliser les deux sorties comme les sorties de deux polariseurs perpendiculaires qui seraient utilisés simultanément. L'intégralité de la lumière entrante est ainsi utilisée.

Les moyens de déviation peuvent être avantageusement réalisés sous la forme d'un prisme de Wollaston, dont les axes sont orientés à 45 degrés des polarisations des bras. Ce prisme est un dispositif commercial courant qui est habituellement utilisé pour séparer angulairement des radiations polarisées linéairement. L'angle de séparation est une caractéristique du prisme de Wollaston et peut être choisie dans une large gamme.

Le montage d'un prisme de Wollaston en sortie d'un interféromètre de Michelson plein champ permet ainsi de réaliser plusieurs simultanées des interférogrammes à des états de phase différents. Il rend particulièrement simple et robuste l'extraction des informations d'amplitude et de phase des rayonnements issus de l'interféromètre. Le dispositif selon l'invention s'applique particulièrement bien au cas de la tomographie par OCT plein champ sur des échantillons mécaniquement instables à une échelle interférométrique.

Lorsque l'interféromètre de Michelson est mis en oeuvre dans un système de tomographie OCT, l'extraction du contraste interférométrique des données se fait, compte tenu de la très faible valeur de ce contraste, par l'empli de techniques de modulation associées à des méthodes de détection synchrone. Deux à quatre mesures, selon le cas, obtenues pour des différences de marche différant de $\lambda/2$ ou $\lambda/4$ le cas échéant, sont ainsi réalisées. Ces mesures sont, par le principe même de la modulation, obtenues à des dates différentes. Garantir la cohérence de ces mesures impose de pouvoir garantir l'invariance de la différence de marche (hors la modulation recherchée) constante. A $\lambda = 780$ nm, pour une fréquence d'échantillonnage de 100hz, par exemple, garantir la différence de marche à $\lambda/4$ près, s'exprime en imposant une variation de la différence de marche inférieure à $V=100*\lambda/4=2\mu$m/sec. Une telle contrainte rend toute mesure in vivo quasi impossible.

Un second objectif de la présente invention est de résoudre ce problème relatif à la variabilité de la différence de marche. Cet objectif est atteint avec l'emploi d'un prisme de Wollaston qui permet d'obtenir au moins deux mesures strictement simultanées et en opposition de phase. En effet, si la première projection mène à la quantité :

$$Ia=I1+I2+2.\sqrt{(I1.I2)}.\cos(\varphi) \qquad (I)$$

où $\varphi$ est le déphasage relatif des deux voies, alors la seconde projection conduit à :

$$Ia=I1+I2-2.\sqrt{(I1.I2)}.\cos(\varphi) \qquad (II)$$

Ce qui s'écrit aussi :

$$Ib=I1+I2+2.\sqrt{(I1.I2)}.\cos(\varphi+\pi) \qquad (III)$$

On dispose donc bien simultanément de deux réalisations du système d'interférence à deux différences de marche séparées de $\lambda/2$.

Un autre problème rencontré dans un interféromètre de Michelson concerne la disponibilité de quatre mesures par modulation. En effet, l'obtention de quatre mesures pose encore plus de problèmes que l'obtention de deux mesures par modulation, toujours pour des raisons de variabilité de la différence de marche.

Ce problème de disponibilité de quatre mesures est résolu en séparant en deux le faisceau par une lame séparatrice simple non polarisante. Dans l'un des deux faisceaux produits, un retard supplémentaire de $\lambda/4$ est imposé entre les polarisations issues des deux bras de l'interféromètre. Une lame quart d'onde permet d'imposer ce retard. Les deux faisceaux ainsi conditionnés sont ensuite réintroduits ensemble et avec un petit angle, dans le prisme de Wollaston de telle sorte qu'en sortie de ce dernier, on dispose alors de quatre faisceaux avec les intensités suivantes :

$$Ia=I1+I2+2.\sqrt{(I1.I2)}.\cos(\varphi) \qquad (IV)$$
$$Ia=I1+I2+2.\sqrt{(I1.I2)}.\cos(\varphi+\pi/2)$$
$$Ia=I1+I2+2.\sqrt{(I1.I2)}.\cos(\varphi+\pi)$$
$$Ia=I1+I2+2.\sqrt{(I1.I2)}.\cos(\varphi+3\pi/2)$$

Les interférogrammes doivent être consistants, c'est à dire que le déphasage impose (0, π/2, π, 3 π/2) doit être constant dans le champ.

A cette fin le prisme de Wollaston est utilisé dans un plan pupille. Tous les points du champ voient ainsi la même portion du prisme, ce qui garantit la condition fixée de constante du déphasage dans le champ.

Il est à noter que les équations (IV) précitées ne sont valables que si l'angle de direction des polarisations est rigoureusement égal à 45 degrés. Pour atteindre cet objectif, on dispose une lame demi-onde précédant le prisme de Wollaston. Cette lame demi-onde permet d'orienter arbitrairement, mais ensemble, les polarisations des quatre faisceaux incidents par rapport aux axes propres du prisme de Wollaston. Le prisme de Wollaston est ainsi précisément aligné par rapport au détecteur, par exemple selon la direction de séparation parallèle aux lignes du détecteur, sans hypothèse sur l'alignement précis des polarisations incidentes par rapport à lui. La lame demi-onde joue un rôle d'interface entre le montage en amont du prisme de Wollaston et le montage en aval.

Les fréquences de modulation souhaitées sont généralement assez élevées, ce qui impose des temps de pose et de lecture assez courts. Ce problème de cadence image et de temps de pose est résolu avec une simultanéité des mesures qui supprime alors toute nécessité de rapidité pour le détecteur. La brièveté de la pose, le cas échéant, peut être assurée par la brièveté de l'éclairement, par exemple un flash, sans que ni l'intégration du détecteur, ni sa lecture, ne soient brèves ou rapides.

Suivant un autre aspect de l'invention, il est proposé un système d'examen de l'oeil par tomographie in vivo, comprenant :

- un interféromètre de Michelson, réalisant un montage d'OCT plein champ,
- des moyens d'optique adaptative, disposés entre l'interféromètre et un oeil à examiner, réalisant la correction des fronts d'onde en provenance de l'oeil mais aussi à destination de l'oeil, et
- des moyens de détection, disposé en aval de l'interféromètre, permettant sans modulation ni détection synchrone, de réaliser la mesure interférométrique selon le principe de l'OCT,

caractérisé en ce qu'il comprend en outre un dispositif pour mesurer le contraste des franges dans un interféromètre de Michelson en plein champ, ce dispositif comprenant des moyens pour dévier deux polarisations perpendiculaires entrantes dans deux directions émergentes différentes.

Ce système d'examen selon l'invention peut en outre avantageusement comprendre un dispositif de visée comprenant au moins une cible mobile présentant une forme et une trajectoire programmable, cette au moins une cible étant affichée sur un écran approprié, visible des deux yeux, pendant la durée de l'examen.

Il est à noter que, bien qu'il ait déjà été proposé d'utilisé un prisme de Wollaston dans un interféromètre de Michelson fonctionnant en OCT, comme par exemple dans le document US 7,079,255 ou dans la publication Hitzenberger et al (1999) : « Differential phase contrast in optical coherence tomography », ce prisme est utilisé dans une partie de l'interféromètre qui est différente de celle de l'invention, et pour un obtenir un effet différent.

Ainsi, le document Hitzenberger et al. décrit un interféromètre de Michelson utilisé en OCT comprenant un prisme de Wollaston dans son bras de mesure, c'est-à-dire celui qui va vers l'objet à examiner. Il s'agit d'obtenir une mesure sur deux rayons parallèles frappant l'échantillon en même temps en deux endroits distincts adjacents par exemple pour observer une bulle dans un film de colle.

De son côté, et pour un système d'OCT fonctionnant par balayage et non en plein champ, le document US 7,079,255 propose d'insérer dans le bras de mesure un dispositif qui dédouble le faisceau lumineux en deux faisceaux de géométries différentes à l'intérieur de ce bras de mesure avant de le projeter vers l'objet à examiner. Ce dispositif de dédoublement peut être un miroir déflecteur à séparation ou un prisme de Wollaston. Ce dédoublement permet d'obtenir un signal d'interférence qui ne représente pas la réflectance d'un point mais une différence de réflectance entre deux points de la zone observée.

Ainsi, ces documents proposent d'utiliser un prisme de Wollaston dans le bras de mesure pour produire des informations sur un ensemble de deux points différents.

De façon différente, l'invention propose d'insérer des moyens pour dévier deux polarisations perpendiculaires dans le bras de sortie pour améliorer le contraste obtenu pour un point donné de la zone observée.

D'autres avantages et caractéristiques de l'invention apparaîtront à l'examen de la description détaillée d'un mode de mise en oeuvre nullement limitatif, et des dessins annexés sur lesquels :

- la figure 1 est un schéma synoptique d'un système de tomographie OCT plein champ représentatif de l'art antérieur ;
- la figure 2 illustre le principe d'un prisme de Wollaston mis en oeuvre dans un dispositif d'amélioration selon l'invention ;
- la figure 3 illustre schématiquement une première configuration, de mesure en deux phases, d'un dispositif d'amélioration de contraste selon l'invention,
- la figure 4 illustre schématiquement une seconde configuration, de mesure en quatre phases, d'un dispositif d'amélioration de contraste selon l'invention ; et
- la figure 5 illustre schématiquement un exemple pratique de réalisation d'un système de tomographie in vivo intégrant un dispositif d'amélioration de contraste selon l'invention.
- la figure 6 est un schéma d'un autre exemple de réalisation d'un système de tomographie in vivo selon l'invention.

On va maintenant tout d'abord décrire, en référence aux figures 2 et 3, le principe de la mesure en deux phases mis en oeuvre dans le dispositif selon l'invention.

Si l'on nomme $a_1$ (resp. $a_2$), l'amplitude du champ électrique en provenance du bras de référence (resp. mesure) de l'interféromètre de Michelson, la projection sur l'un des axes du prisme de Wollaston a pour amplitude :

$$Av = \sqrt{2}/2(a_1 + a_2)$$

tandis que la projection sur l'autre axe du prisme de Wollaston aura pour amplitude :

$$Ah = \sqrt{2}/2(a_1 - a_2)$$

Les deux projections sont séparées angulairement de l'angle caractéristique du prisme de Wollaston. Focalisées par l'objectif de chambre, elles produisent deux images interférométriques du champ juxtaposées, dont les énergies ont pour expression :

$$Iv = |Av|^2 \text{ et } Ih = |Ah|^2$$

soit

$$Iv = 1/2(a_1^2 + a_2^2 + 2a_1 . a_2 . \cos(\varphi))$$

et

$$Ih = 1/2(a_1^2 + a_2^2 - 2a_1 . a_2 . \cos(\varphi))$$

où $\varphi$ est la différence de phase entre les deux rayonnements $a_1$ et $a_2$.
La seconde égalité peut aussi s'écrire :

$$Ih = 1/2(a_1^2 + a_2^2 + 2a_1 . a_2 . \cos(\varphi + \pi))$$

Ainsi, on obtient simultanément deux images correspondent à deux états de l'interférogramme en opposition de phase. La durée de la pose (ou de l'illumination de l'échantillon) peut être rendue arbitrairement courte, dans la limite de la détection, pour garantir le « gel » de l'information interférométrique. Néanmoins, la simultanéité des deux mesures, donc le déphasage de π, n'est jamais remise en cause. Il faut également noter que ce déphasage est obtenu mathématiquement, par construction, et est totalement achromatique.
En réalité, la quantité $a_2$ doit être vue comme la superposition d'un rayonnement d'amplitude $a_{20}$ qui n'interfère pas avec $a_1$ faute d'une différence de marche suffisamment petite, et d'un rayonnement d'amplitude $a_{2*}$ réfléchi avec une différence de marche inférieure à la longueur de cohérence de la source utilisée, qui lui, interfère. On a donc :

$$Iv = 1/2(a_1^2 + a_2^2 + 2a_1 . a_{2*} . \cos(\varphi))$$

$$Ih = 1/2(a_1^2 + a_2^2 + 2a_1 . a_{2*} . \cos(\varphi + \pi))$$

De la différence entre Ih et Iv, on tire la quantité réellement module:

$$Im = 2a_1 . a_{2*} . \cos(\varphi)$$

C'est cette même quantité que l'on obtient par détection synchrone, mais alors au prix d'une dichotomie temporelle de la mesure, puisque la moitié du temps de pose doit être consacrée à chaque mesure, à $\varphi$ et $\varphi + \pi$.
Outre la simultanéité, cette méthode offre donc un avantage photométrique qui trouve son origine dans le fait que les deux projections possibles à 45 degrés sont traitées. Toute l'énergie disponible est utilisée. Toutefois, la quantité Im ne sépare pas $a_{2*}$ de $\varphi$, en considérant $a_1$ connu. Il reste une ambiguïté entre l'amplitude et le cosinus de la phase.

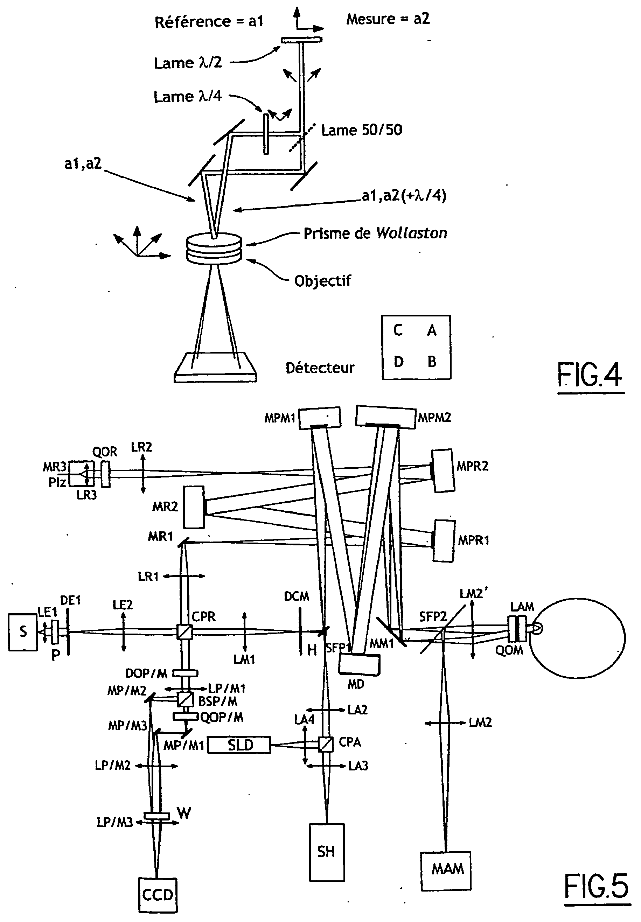

Dans une seconde configuration du dispositif selon l'invention illustrée par la figure 4, on effectue une mesure selon quatre phases. On prélève avant le prisme de Wollaston 50% de l'énergie via une lame semi-réfléchissante. Sur le faisceau dévié, où l'on trouve les rayonnements des deux bras encore polarisés perpendiculairement, on installe une lame quart d'onde dont les axes sont alignés avec les deux polarisations incidentes. On impose ainsi un déphasage supplémentaire de n/2 entre les deux bras.

Il suffit alors d'injecter le faisceau dans le même prisme de Wollaston, mais avec un petit angle par rapport au faisceau non dévié, qui peut d'ailleurs être choisi égal à l'angle caractéristique du prisme de Wollaston, pour obtenir en sortie quatre faisceaux angulairement séparés. Pour des raisons pratiques, on peut faire précéder l'ensemble d'une lame demi-onde dont la fonction est de préparer l'orientation de toutes les polarisations.

Les amplitudes du champ dans les quatre faisceaux sont :

$$A_A = 1/2(a_1 + a_2)$$

$$A_B = 1/2(a_1 - a_2)$$

$$A_C = 1/2(a_1 + a_2[\Pi/2])$$

$$A_D = 1/2(a_1 - a_2[\Pi/2])$$

où $a_2[\Pi/2]$ représente l'amplitude du rayonnement $a_2$ déphasé de n/2.

A ces quatre amplitudes correspondent quatre intensités mesurées :

$$I_A = 1/4(a_1^2 + a_2^2 + 2a_1.a_{2*}.\cos(\varphi))$$

$$I_B = 1/4(a_1^2 + a_2^2 - 2a_1.a_{2*}.\cos(\varphi))$$

$$I_C = 1/4(a_1^2 + a_2^2 + 2a_1.a_{2*}.\cos(\varphi + \Pi/2))$$

$$I_D = 1/4(a_1^2 + a_2^2 - 2a_1.a_{2*}.\cos(\varphi + \Pi/2))$$

Soit encore:

$$I_A = 1/4(a_1^2 + a_2^2 + 2a_1.a_{2*}.\cos(\varphi))$$

$$I_B = 1/4(a_1^2 + a_2^2 - 2a_1.a_{2*}.\cos(\varphi))$$

$$I_C = 1/4(a_1^2 + a_2^2 + 2a_1.a_{2*}.\cos(\varphi + \Pi/2))$$

$$I_D = 1/4(a_1^2 + a_2^2 - 2a_1.a_{2*}.\cos(\varphi + \Pi/2))$$

On a bien cette fois quatre images interférométriques simultanées du champ, correspondent à quatre états de phases différents de l'interférogramme, desquelles on peut tirer classiquement les deux quantités :

$$Im_1 = I_A - I_B = a_1.a_{2*}.cos(\varphi)$$

$$Im_2 = I_C - I_D = a_1.a_{2*}.cos(\varphi + \pi/2) = a_1.a_{2*}.sin(\varphi)$$

Ce qui permet d'éliminer la phase par somme des carrés:

$$I = Im_1^2 + Im_2^2 = a_1^2.a_{2*}^2 = I_1.I_{2*}$$

où $I_1$ et $I_{2*}$ sont les intensités retournées par le bras de référence de l'interféromètre de Michelson et l'échantillon, à la profondeur sélectionnée par la longueur du bras de référence. Du rapport de $Im_1$ et $Im_2$, on peut tirer $tg(\varphi)$ indépendamment de $I_{2*}$.

Si l'emploi d'un prisme de Wollaston donne accès à des mesures simultanées de l'interférogramme à des états de phase différents, ces mesures sont néanmoins réalisées avec des détecteurs ou des pixels du capteur CCD différents. Un étalonnage soigné de la sensibilité et du biais propre de chaque pixel doit donc être effectué pour écarter toute possibilité de biais dans le calcul des différences $Im_1$ et $Im_2$ et donc de la somme de leur carré.

On va maintenant décrire, en référence à la figure 5, un exemple pratique de réalisation d'un système de tomographie in vivo selon l'invention intégrant un dispositif de mesure de contraste interférométrique. Ce système comprend un interféromètre, de type Michelson plein champ, comportant un bras de mesure prévu pour illuminer l'oeil et collecter la lumière renvoyée, et un bras de référence prévu pour illuminer un miroir mobile permettant l'exploration en profondeur du tissu rétinien.

L'interféromètre est utilisé en lumière polarisée de façon rectiligne et perpendiculaire dans les deux bras. La source de lumière S est une diode à faible longueur de cohérence temporelle (par exemple, 12 $\mu$m), dont le spectre est centré sur 780 nm. Elle confère par principe au système de tomographie in vivo une résolution axiale égale à la moitié de la longueur de cohérence divisée par l'indice de réfraction du milieu.

Cette source de lumière S peut être pulsée. Dans ce cas, elle est alors synchronisée avec la prise d'image et la correction adaptative. Le faisceau est limité par un diaphragme de champ correspondant à 1 degré dans le champ de vue de l'oeil (300 $\mu$m sur la rétine) et un diaphragme pupillaire correspondant à une ouverture de 7 mm sur un oeil dilaté.

Un polariseur d'entrée P permet l'équilibrage optimal des flux injectés dans les deux bras de l'interféromètre.

Les deux bras présentent une configuration dite de Gauss, afocale, qui permet le transport des pupilles, d'une part, et la matérialisation d'une image intermédiaire du champ où un diaphragme bloque une grande part du reflet cornéen, d'autre part. Des lames quart d'onde assurent par la rotation de la polarisation de la seule lumière renvoyée par l'oeil, et le miroir mobile, un filtrage efficace des réflexions parasités dans le système de tomographie in vivo selon l'invention.

Afin de conserver l'égalité des chemins optiques dans les deux bras, avec le même transport des pupilles et du champ, le bras de référence est similaire au bras de mesure, mais avec un optique statique.

On va maintenant décrire la voie de détection du système de tomographie in vivo selon l'invention. Les deux faisceaux sur le bras de sortie sont encore polarisés perpendiculairement, et ils n'interfèrent que s'ils sont projetés sur une direction commune. Un prisme de Wollaston W a pour fonction de projeter simultanément les deux rayonnements sur deux directions d'analyse perpendiculaires. On peut alors effectuer une mesure simultanée de l'intensité après interférence dans deux états d'interférence en opposition, sans modulation ni détection synchrone, sur un détecteur bidimensionnel unique. L'adjonction d'une lame quart d'onde, après division du faisceau, permet d'accéder à deux mesures supplémentaires, levant ainsi toute ambiguïté entre amplitude et phase des franges. Une lame demi onde à l'entrée de la voie de détection permet d'orienter convenablement les polarisations incidentes.

Le prisme de Wollaston est placé dans un plan pupillaire, donc conjugué du cube séparateur de l'interféromètre de Michelson. L'angle de séparation du prisme de Wollaston est choisi en fonction du champ à observer. La longueur focale de l'objectif final détermine le pas d'échantillonnage des quatre images.

Le détecteur est du type CCD, avec une cadence d'image est supérieure à 30 images par seconde. Ce détecteur est associé à un calculateur dédié (non représenté) dans lequel es réalisé le traitement numérique des images : extraction des quatre mesures, étalonnage, calcul de l'amplitude des franges.

La correction adaptative des fronts d'onde est réalisée en amont de l'interféromètre, donc dans le bras de mesure. Chaque point de la source S voit ainsi son image sur la rétine corrigée des aberrations, et l'image en retour est également corrigée. L'amplitude des franges est alors maximale.

Le sous-ensemble d'optique adaptative comprend un miroir déformable MD. La mesure de front d'onde est faite par un analyseur SH de type Shack-Hartmann sur le faisceau de retour d'un spot lumineux lui-même imagé sur la rétine via le miroir déformable MD. La longueur d'onde d'analyse est de 820 nm. L'éclairage est continu et fourni par une diode SLD superluminescente temporellement incohérente. Le dimensionnement de l'analyseur correspond à une optimisation entre sensibilité photométrique et échantillonnage du front d'onde. La cadence de rafraîchissement de la commande du miroir déformable MD peut atteindre 150 Hz. Un calculateur dédié (non représenté) gère la boucle d'optique adaptative. La commande est toutefois synchronisée pour geler la forme du miroir pendant la mesure interférométrique.

Un contrôle approprié de la focalisation de la voie d'analyse, au moyen d'une lentille LA2, permet d'adapter la distance de focalisation à la couche sélectionnée par l'interféromètre. Cette disposition est capitale pour conserver un contraste optimal à toute profondeur.

Le miroir déformable MD est conjugué de la pupille du système et de l'oeil. Le champ du système est défini par le diaphragme de champ DCM d'entrée du système. Il est choisi égal à 1 degré, soit moins que le champ d'isoplanétisme de l'oeil, ce qui garantit la validité de la correction adaptative dans le champ sur la seule mesure de front d'onde réalisée à partir du spot, au centre du champ. De plus, la rotation du miroir déformable MD permet de choisir l'angle d'arrivée du faisceau dans l'oeil, donc la portion de rétine étudiée.

La mesure OCT suppose l'égalité des chemins optiques entre les deux bras de l'interféromètre de Michelson, à la longueur de cohérence de la source près. Elle suppose également une mise au point optimale sur la profondeur qui correspond à cette égalité. Traditionnellement, la limitation du diamètre du faisceau confère à l'oeil une profondeur de champ très grande qui dispense d'une quelconque re-mise au point.

Quand le système est utilisé à pleine ouverture (typiquement F/3), la profondeur de champ diminue rapidement, typiquement 30 $\mu$m. Le balayage en Z de l'OCT peut sortir rapidement de cet intervalle, au-delà duquel le contraste interférométrique diminue. On peut considérer cela comme un effet d'aberration de pur défocus.

On peut remédier à ce problème en dotant l'analyseur de surface d'onde d'un dispositif permettant de régler sa propre focalisation, par exemple avec un réglage mécanique. Une modification arbitraire de cette focalisation, force, via la boucle d'optique adaptative, le miroir déformable à adopter une courbure supplémentaire, conjuguant source d'entrée et détecteur avec un point plus ou moins profond dans la rétine. La commande de cette focalisation doit être synchronisée avec le balayage en Z de l'OCT.

Il est aussi possible de commander l'analyseur pour le forcer à travailler en défocalisé. Certains analyseurs évolués, par exemple les modèles de la société Imagine Optic, sont en effet capables de travailler en défocalisé avec de bons résultats.

Une solution alternative à une réelle défocalisation de l'analyseur peut consister à ajouter un terme de focus pur dans la commande du miroir, quelle que soit la mesure de l'analyseur. Cet artifice est couramment utilisé en optique adaptative. Avec un analyseur de type Shack-Hartmann, on modifie simplement le tableau dit « des pentes de référence », ce qui force le système à converger vers une commande arbitrairement modifiée.

L'adjonction de verres correcteurs de la vue du sujet, donc des bas ordres d'aberrations géométriques tels que le focus ou l'astigmatisme, juste devant l'oeil, permet de relâcher les exigences sur la course du miroir déformable MD, et garantit également une meilleure visée. Un système correcteur adaptatif par transmission peut être utilisé de préférence à des verres fixes pour une correction optimale.

Un système de visée collaboratif ou actif est installé en amont de l'ensemble. Ce système de visée, qui comprend une mire active MAM, présente au sujet l'image d'un point lumineux s'écartant périodiquement de l'axe de visée recherché. Le patient est alors invité à suivre tous les mouvements de cette image. Chaque fois que l'image revient sur l'axe, et après un temps de latence ajustable, une série de mesures interférométriques est réalisée. Le déplacement périodique du regard permet d'obtenir du patient une meilleure capacité de fixation quand il vise l'axe recherché. L'amplitude et la fréquence sont adaptables au sujet et aux mesures entreprises. Pour des raisons de commodité, la mire peut être réalisée avec un simple ordinateur de bureau sur lequel un point lumineux est affiché et déplacé. La mire active MAM, l'optique adaptative, la source S et la prise d'image sont synchronisées.

Dans l'exemple pratique de réalisation illustré par la figure 5, le système de tomographie in vivo selon l'invention est relativement compact, moins de 1,2 m de côté. Une part importante de la contrainte de taille vient du diamètre du miroir déformable MD qui fixe en partie la longueur focale des paraboles hors axe. L'emploi de micro-miroirs diminuerait évidemment toutes les dimensions du système.

Le système de détection, avec sa division en deux faisceaux, est réalisé ici avec des composants discrets. Il est envisageable de faire réaliser et d'utiliser des composants intégrés réunissant les fonctions de séparation, repliement, voire, retard des faisceaux.

En ce qui concerne l'analyse de front d'onde, on notera que l'installation de la source de référence SLD en amont du miroir déformable MD permet une qualité optimale de mesure des aberrations donc de leur compensation, puisque l'image de référence matérialisée dans l'oeil bénéficie dans ce cas de la correction adaptative. Cette optimisation reste vraie quelle que soit la focalisation, la source SLD étant en amont du système de contrôle de mise au point. Enfin, l'emploi d'un cube polarisant CPA (fig. 4) permet là encore d'utiliser tous les photons issus de l'oeil. La mesure du front d'onde est donc faite dans de bonnes conditions.

Cependant, il est à noter qu'une conjugaison optique latérale très précise est requise entre la source de référence SLD et l'entrée de l'analyseur SH. Dans le cas où cette conjugaison est insuffisante, une tentative de compensation par la boucle d'optique adaptative d'une erreur de conjugaison peut mener à une divergence de l'asservissement.

Une variante de l'invention, illustrée en figure 5, permet plus de simplicité dans le système en diminuant ce risque d'instabilité de la boucle d'optique adaptative. Dans cette variante, la source de référence SLD est positionnée plus près de l'oeil dans le trajet optique, en particulier après l'optique adaptative (à l'aller) et par exemple avant un compensateur de biréfringence, comme un compensateur de Soleil-Babinet CBC, ou juste avant l'oeil. A défaut de bénéficier d'une tache image optimale au fond de l'oeil, le système gagne alors en stabilité de fonctionnement.

Le miroir déformable utilisé dans l'optique adaptive peut être par exemple un miroir de diamètre 50 mm à 31 éléments de la société CILAS. Cependant, les performances et/ou la compacité du dispositif peuvent être améliorées en utilisant un modèle plus performant et/ou plus compact tel que le miroir déformable de diamètre 15 mm à 52 éléments développé au Laboratoire d'Astrophysique de l'Observatoire de Grenoble, en particulier du fait de sa compacité et d'une course plus importante dans les mouvements d'adaptation.

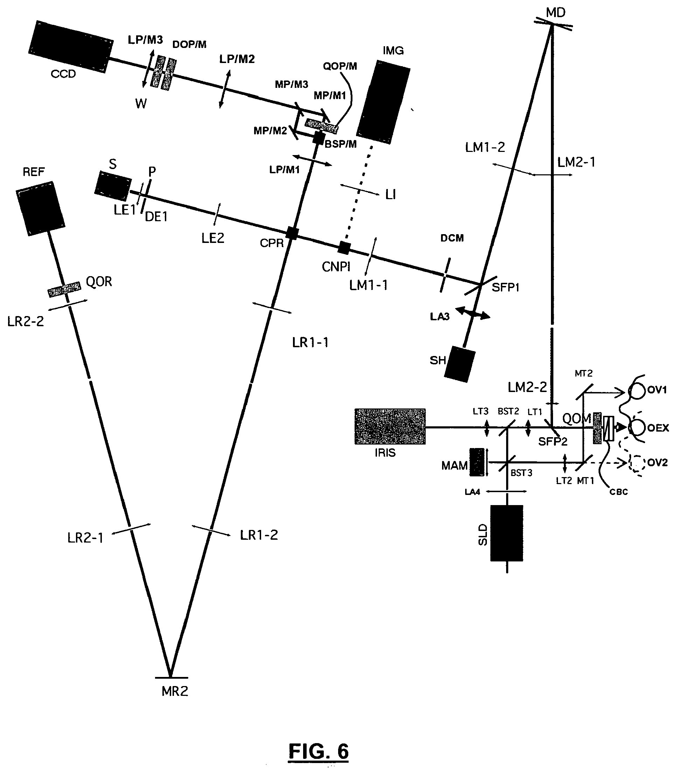

Dans l'exemple illustré en figure 6, les miroirs de repliement du bras de mesure MPM1, MPM2 et ceux du bras de référence MPR1, MPR2 ont été supprimés. Le trajet optique du bras de mesure comprend un doublet de deux lentilles LM1-1 et LM1-2 d'un côté du miroir déformable MD, et un autre doublet de deux lentilles LM2-1 et LM2-2 de l'autre côté de ce miroir déformable. De la même façon, le trajet optique du bras de référence comprend un doublet de deux lentilles LR1-1 et LR1-2 d'un côté du miroir de référence MR, et un autre doublet de deux lentilles LR2-1 et LR2-2 de l'autre côté de ce miroir de référence.

L'utilisation de lentilles plutôt que de miroirs peut être plus économique et permettre de meilleures performances, en particulier du fait du coût et des aberrations optiques de ce type de miroir, qui sont typiquement des miroirs paraboliques hors axes.

La combinaison d'un tel montage dans l'axe, avec un miroir déformable plus petit permet d'obtenir un système plus performant, plus simple, ou plus économique, tout en conservant un encombrement limité.

Ainsi qu'illustré en figure 6, le système peut en outre comprendre des moyens d'imagerie classique, comme une caméra IMG, permettant d'associer les mesures interférométriques avec une imagerie simple des zones examinées, par exemple pour faciliter l'exploration et la sélection des zones à examiner.

Placé directement en sortie (au retour) du bras de mesure, donc juste avant le cube polarisant CPR de l'interféromètre, un second cube polarisant CNPI permet de dévier le faisceau de retour vers une caméra d'imagerie IMG disposant de ses propres moyens de focalisation LI de l'image. Sur cette voie, une image directe de la zone rétinienne visée sera observable. On peut en particulier agencer le bras de mesure et cette voie additionnelle de sorte qu'ils procurent un champ d'observation plus large que le mode interférométrique, dont le champ est limité en particulier par la technique de mesure de contraste interférométrique en elle-même.

Du fait de sa faible longueur de cohérence, la source d'entrée S présente un spectre de type polychromatique. Dans un montage OCT typique ce spectre est en général relativement étroit, par exemple d'une largeur de l'ordre de 50 nanomètres, mais pas forcément négligeable.

Ce spectre polychromatique peut causer une dégradation des performances, en particulier en entraînant une dispersion des différences de marche du fait du caractère dispersif du milieu oculaire, ce qui conduit à une dégradation de la résolution axiale du dispositif. Pour éviter ou limiter ces dégradations, le système peut comprendre des moyens de compensation situés dans le bras de référence.

De plus, le caractère dispersif des milieux oculaires se traduit également par une variation de la focale de l'oeil avec la longueur d'onde, entraînant aussi une dégradation de la résolution axiale. Pour éviter ou limiter ces dégradations, le système peut alors comprendre des moyens de compensation situés par exemple dans le bras de mesure. En particulier, ces moyens peuvent compenser un chromatisme focal qui représente environ 400 micromètres entre le rouge et le bleu, par exemple en remplaçant le collimateur LM2-2 situé juste devant l'oeil par un doublet au chromatisme volontairement choisi opposé à celui de l'oeil. Ces moyens peuvent également compenser les différences de chemin optique dues à la dispersion chromatique, par exemple en insérant une cuve d'eau dans le bras de référence d'une taille dépendant et/ou réglable selon la taille ou les caractéristiques de l'oeil à examiner. Une telle cuve peut être d'une dimension de l'ordre de 24 mm, longueur moyenne d'un oeil humain.

Avec une source de 12 micromètres de longueur de cohérence et de 50 nanomètres de largeur de spectre, l'utilisation de ces moyens de compensation peut permettre d'améliorer la résolution axiale en la ramenant d'une valeur d'environ 6 micromètres à une valeur d'environ 4 micromètres.

Pour augmenter les performances, en particulier en termes de résolution axiale, le système peut également utiliser comme source d'entrée de l'interféromètre une illumination polychromatique à spectre plus large, par exemple en lumière blanche. Dans ce cas, l'augmentation de performance procurée par ces moyens de compensation sera beaucoup plus importante.

L'invention peut en particulier être mise en oeuvre pour réaliser ou compléter un dispositif d'imagerie rétinienne, ou de topographie cornéenne, ou de mesure d'un film de larmes.

Bien sûr, l'invention n'est pas limitée aux exemples qui viennent d'être décrits et de nombreux aménagements peuvent être apportés à ces exemples sans sortir du cadre de l'invention telle que revendiquée.

## Revendications

1. Dispositif pour mesurer le contraste de franges, combiné avec un interféromètre de Michelson en plein champ en lumière polarisée comprenant au moins un bras de référence et un bras de mesure avec des polarisations perpendiculaires coopérant avec un bras de sortie pour réaliser un système de tomographie optique cohérente (OCT), comprenant dans le bras de sortie des moyens (W) pour dévier deux polarisations perpendiculaires entrantes (a1 et a2 fig.3 ; a1,a2 et a1,a2($\lambda$/4) fig.4) dans deux directions émergeantes (Av et Ah fig.3 ; A, B, C et D fig.4) différentes et pour une obtention simultanée d'interférogrammes dans différents états de phase.

2. Dispositif selon la revendication 1, tel que les moyens de déviation comprennent un prisme de Wollaston (W).

3. Dispositif selon la revendication 2, tel qu'il est agencé pour réaliser des mesures pour des différences de marche différant de $\lambda$/2 ou $\lambda$/4.

4. Dispositif selon la revendication 3, tel qu'il est agencé pour obtenir au moins deux mesures, strictement simultanées et en opposition de phase.

5. Dispositif selon l'une des revendications 2 à 4, tel qu'il est agencé pour réaliser quatre mesures (A, B, C et D fig. 4), et en ce qu'il comprend en outre des moyens ("lame 50/50" fig.4, BSP/M fig.5 et fig.6) pour séparer un faisceau entrant dans le bras de sortie en au moins deux faisceaux séparés, des moyens ("lame $\lambda$/4" fig.4, QOP/M fig.5 et fig.6) pour générer, dans l'un de ces deux faisceaux séparés, un retard supplémentaire de $\lambda$/4 entre les polarisations issues du bras de mesure et du bras de référence de l'interféromètre, et des moyens (MP/M1, MP/M2, MP/M3 fig. 5 et fig.6) pour réintroduire ensemble les deux faisceaux ainsi conditionnés dans le prisme de Wollaston de (W) telle sorte qu'en sortie de ce dernier, on dispose alors de quatre faisceaux (A, B, C, D fig.4).

6. Dispositif selon la revendication 5, tel que les moyens séparateurs comprennent une lame séparatrice simple non polarisante (BSP/M).

7. Dispositif selon l'une des revendications 5 ou 6, tel que les moyens retardateurs comprennent une lame quart d'onde (QOP/M).

8. Dispositif selon l'une des revendications 5 à 7, tel que le prisme de Wollaston (W) est disposé dans un plan pupille.

9. Dispositif selon l'une des revendications 5 à 8, tel qu'il comprend en outre des moyens (DOP/M fig.5) pour orienter arbitrairement les polarisations des quatre faisceaux incidents par rapport aux axes propres du prisme de Wollaston (W).

10. Dispositif selon la revendication 9, tel que les moyens d'orientation comprennent une lame demi-onde (DOP/M) précédant le prisme de Wollaston (W).

11. Procédé pour mesurer le contraste des franges dans un interféromètre de Michelson en plein champ en lumière polarisée comprenant au moins un bras de référence et un bras de mesure avec des polarisations perpendiculaires coopérant avec un bras de sortie pour réaliser un système de tomographie optique cohérente (OCT), le procédé comprenant une déviation de deux polarisations perpendiculaires entrantes dans deux directions émergeantes différentes et pour une obtention simultanée d'interférogrammes dans différents états de phase, au moyen d'un prisme de Wollaston (W) situé dans ledit bras de sortie.

12. Procédé selon la revendication 11, tel qu'il comprend des mesures pour des différences de marche différant de $\lambda$/2 ou $\lambda$/4.

13. Procédé selon la revendication 12, tel qu'il comprend au moins deux mesures strictement simultanées et en opposition de phase.

14. Procédé selon l'une des revendications 11 à 13, tel qu'il comprend quatre mesures, une séparation en deux d'un faisceau entrant dans le bras de sortie, une génération, dans l'un des deux faisceaux produits, d'un retard supplémentaire de À/4 entre les polarisations issues du bras de mesure et du bras de référence de l'interféromètre, et une réintroduction des deux faisceaux ainsi conditionnés dans le prisme de Wollaston (W) de telle sorte qu'en sortie de ce dernier, on dispose alors de quatre faisceaux.

15. Procédé selon la revendication 14, tel qu'il comprend en outre une orientation arbitraire des polarisations des quatre faisceaux incidents par rapport aux axes propres du prisme de Wollaston.

16. Procédé selon la revendication 15, tel que les mesures sur les quatre faisceaux sont réalisées simultanément.

17. Procédé selon l'une des revendications 1 à 16, tel qu'il comprend, dans le bras de mesure, une compensation des effets du chromatisme focal de l'oeil.

18. Procédé selon l'une des revendications 1 à 17, tel qu'il comprend, dans le bras de référence, des moyens de compensation de la dispersion des différences de marche.

19. Procédé selon l'une des revendications 1 à 18, tel qu'il comprend une commande de l'analyseur de front d'onde (SH) l'obligeant à travailler en défocalisé.

20. Système d'examen de l'oeil par tomographie in vivo, comprenant :

   - un interféromètre de Michelson, comprenant au moins un bras de mesure et un bras de référence coopérant avec un bras de sortie pour réaliser un montage d'OCT plein champ,
   - des moyens d'optique adaptative, disposés entre le bras de mesure de l'interféromètre et un oeil à examiner ou au sein dudit bras de mesure, réalisant la correction des fronts d'onde en provenance de l'oeil mais aussi à destination de l'oeil, et
   - des moyens de détection, disposé en aval de l'interféromètre ou au sein de son bras de sortie, permettant de réaliser la mesure interférométrique selon le principe de la tomographie optique cohérente (OCT), tel qu'il comprend en outre un dispositif selon l'une quelconque des revendications 1 à 10.

21. Système d'examen de l'oeil selon la revendication 20, tel qu'il comprend en outre un dispositif de visée comprenant au moins une cible mobile présentant une forme et une trajectoire programmable, ladite cible étant affichée sur un écran approprié, visible des deux yeux, pendant la durée de l'examen.

22. Système selon l'une des revendications 20 ou 21, tel que la source de référence (SLD) est insérée dans le chemin optique entre les moyens d'optique adaptative (MD) et l'oeil à examiner (OEX).

23. Système selon l'une des revendications 20 à 22, qu'il comprend, dans le bras de mesure, des moyens de compensation des effets du chromatisme focal de l'oeil.

24. Système selon l'une des revendications 20 à 23, tel qu'il comprend dans le bras de référence des moyens de compensation de la dispersion des différences de marche.


**Patentansprüche**

1. Vorrichtung zur Messung des Kontrastes von Interferenzstreifen, in Kombination mit einem Vollfeld-Michelson-Interferometer mit polarisiertem Licht, das mindestens einen Referenzarm und einen Messarm mit zueinander rechtwinkligen Polarisationen umfasst, die mit einem Ausgangsarm zur Erzeugung eines Systems zur optischen Kohärenztomografie (OCT) zusammenwirken, wobei sie im Ausgangsarm Mittel (W) zur Ablenkung zweier eingehenden, zueinander rechtwinkligen Polarisationen (a1 und a2 Fig.3; a1,a2 und a1,a2 ($\lambda$/4) Fig.4) in zwei verschiedene ausfallende Richtungen (Av und Ah Fig.3; A, B, C und D Fig.4) und zur simultanen Erstellung von Interferogrammen in verschiedenen Phasenlagen umfasst.

2. Vorrichtung nach Anspruch 1, wobei die Ablenkungsmittel ein Wollaston-Prisma (W) umfassen.

3. Vorrichtung nach Anspruch 2, wobei sie zur Durchführung von Messungen für um λ/2 oder λ/4 abweichende Gangunterschiede ausgebildet ist.

4. Vorrichtung nach Anspruch 3, wobei sie zur Erlangung mindestens zweier, streng simultanen und gegenphasigen Messungen ausgebildet ist.

5. Vorrichtung nach einem der Ansprüche 2-4, wobei sie zur Durchführung von vier Messungen (A, B, C und D Fig.4) ausgebildet ist und wobei sie außerdem Mittel ("50/50-Platte" Fig.4, BSP/M Fig.5 und Fig.6) zum Aufteilen eines in den Ausgangsarm einfallenden Strahlenbündels in mindestens zwei getrennte Bündel, Mittel ("λ/4-Plättchen" Fig. 4, QOP/M Fig.5 und Fig.6) zur Erzeugung einer zusätzlichen Phasenverzögerung um λ/4 zwischen den aus dem Messarm und dem Referenzarm des Interferometers stammenden Polarisationen in einem dieser beiden getrennten Bündel, sowie Mittel (MP/M1, MP/M2, MP/M3 Fig.5 und Fig.6) zur Wiederzusammenführung der beiden, entsprechend bedingten Strahlenbündel in das Wollaston-Prisma (W), damit am Ausgang des Letzteren vier Strahlenbündel (A, B, C, D Fig.4) nun vorhanden sind, umfasst.

6. Vorrichtung nach Anspruch 5, wobei die aufteilenden Mittel eine einfache, nicht polarisierende Strahlteilerplatte (BSP/M) umfassen.

7. Vorrichtung nach einem der Ansprüche 5 oder 6, wobei die phasenverzögernden Mittel ein Viertelwellenplättchen (QOP/M) umfassen.

8. Vorrichtung nach einem der Ansprüche 5-7, wobei das Wollaston-Prisma (W) in einer Pupillen-Ebene gelagert ist.

9. Vorrichtung nach einem der Ansprüche 5-8, wobei sie außerdem Mittel (DOP/M Fig.5) zur willkürlichen Lenkung der Polarisationen der vier einfallenden Strahlenbündel zu den Eigenachsen des Wollaston-Prismas (W) umfasst.

10. Vorrichtung nach Anspruch 9, wobei die Lenkungsmittel ein dem Wollaston-Prisma (W) vorgelagertes Halbwellenplättchen (DOP/M) umfassen.

11. Verfahren zur Messung des Kontrastes der Interferenzstreifen in einem Vollfeld-Michelson-Interferometer mit polarisiertem Licht, das mindestens einen Referenzarm und einen Messarm mit zueinander rechtwinkligen Polarisationen umfasst, die mit einem Ausgangsarm zur Erzeugung eines Systems zur optischen Kohärenztomografie (OCT) zusammenwirken, wobei das Verfahren eine Ablenkung von zwei einfallenden, zueinander rechtwinkligen Polarisationen in zwei verschiedene, ausfallende Richtungen sowie eine simultane Erstellung von Interferogrammen in verschiedenen Phasenlagen mittels eines in dem Ausgangsarm gelagerten Wollaston-Prismas (W) umfasst.

12. Verfahren nach Anspruch 11, wobei es Messungen für um λ/2 oder λ/4 abweichende Gangunterschiede umfasst.

13. Verfahren nach Anspruch 12, wobei es mindestens zwei streng simultane und gegenphasige Messungen umfasst.

14. Verfahren nach einem der Ansprüche 11-13, wobei es vier Messungen, eine Aufspaltung eines in den Ausgangsarm eingehenden Strahlenbündels in zwei, eine Erzeugung einer zusätzlichen Phasenverzögerung um λ/4 zwischen den aus dem Messarm und dem Referenzarm des Interferometers ausgehenden Polarisationen in einem der zwei erzeugten Strahlenbündel, sowie eine Wiederzusammenführung der beiden, entsprechend bedingten Strahlenbündel in das Wollaston-Prisma (W), damit am Ausgang des Letzteren nun vier Strahlenbündel vorhanden sind, umfasst.

15. Verfahren nach Anspruch 14, wobei es außerdem eine willkürliche Lenkung der Polarisationen der vier einfallenden Strahlenbündel zu den Eigenachsen des Wollaston-Prismas umfasst.

16. Verfahren nach Anspruch 15, wobei die Messungen an den vier Strahlenbündeln simultan erfolgen.

17. Verfahren nach einem der Ansprüche 1-16, wobei es in dem Messarm einen Ausgleich der Effekte des Farblängsfehlers des Auges umfasst.

18. Verfahren nach einem der Ansprüche 1-17, wobei es in dem Referenzarm Mittel zum Ausgleich der Dispersion der Gangunterschiede umfasst.

19. Verfahren nach einem der Ansprüche 1-18, wobei es einen Befehl des Wellenfrontanalysators (SH) umfasst, der es zur Funktionsweise in defokussiertem Modus zwingt.

20. System zur Untersuchung des Auges mittels in-vivo Tomografie, umfassend:

- ein Michelson-Interferometer, mindestens einen Messarm und einen Referenzarm umfassend, mit einem Ausgangsarm zur Erzeugung einer Vollfeld-OCT-Abbildung zusammenwirkend,
- Adaptiv-Optikeinrichtungen, zwischen dem Messarm des Interferometers und einem zu untersuchenden Auge oder innerhalb des Messarms angeordnet, die Korrektur der vom Auge kommenden aber auch zum Augen hin gehenden Wellenfronten ausführend, und
- Erkennungseinrichtungen, dem Interferometer nachgelagert oder innerhalb seines Ausgangsarms angeordnet, die Ausführung der interferometrischen Messung nach dem Prinzip der optischen Kohärenztomografie (OCT) ermöglichend,

wobei es außerdem eine Vorrichtung nach irgendeinem der Ansprüche 1-10 umfasst.

21. System zur Untersuchung des Auges nach Anspruch 20, wobei es außerdem eine Visiervorrichtung umfasst, die mindestens ein bewegliches Ziel mit einer programmierbaren Form und einem programmierbaren Bewegungspfad umfasst, wobei das Ziel auf einem geeigneten, durch beide Augen sichtbaren Bildschirm für die Dauer der Untersuchung angezeigt wird.

22. System nach einem der Ansprüche 20 oder 21, wobei die Referenzquelle (SLD) im optischen Weg zwischen den Adaptiv-Optikeinrichtungen (MD) und dem zu untersuchenden Auge (OEX) überlagert ist.

23. System nach einem der Ansprüche 20-22, wobei es in dem Messarm Mittel zum Ausgleich der Effekte des Farblängsfehlers des Auges umfasst.

24. System nach einem der Ansprüche 20-23, wobei es in dem Referenzarm Mittel zum Ausgleich der Dispersion der Gangunterschiede umfasst.

**Claims**

1. Device for measuring the contrast of fringes, combined with a full-field polarized light Michelson interferometer comprising at least one reference arm and one measurement arm with perpendicular polarizations co-operating with an output arm in order to produce an optical coherence tomography system (OCT), comprising in the output arm means (W) of deflecting two incoming perpendicular polarizations (a1 et a2 fig.3 ; a1,a2 et a1,a2($\lambda$/4) fig.4) in two different emerging directions (Av et Ah fig.3 ; A, B, C et D fig.4) and for a simultaneous production of interferograms with under different phase states.

2. Device according to claim 1, such that the means of deflection comprise a Wollaston prism.

3. Device according to claim 2, such that it is arranged to carry out measurements for path differences differing by $\lambda$/2 or $\lambda$/4.

4. Device according to claim 3, such that it is arranged so as to obtain at least two measurements, strictly simultaneous and in phase opposition.

5. Device according to one of claims 2 to 4, such that it is arranged in order to carry out four measurements (A, B, C et D fig.4), and in that it also comprises means ("lame 50/50" fig.4, BSP/M fig.5 et fig.6) to separate a beam entering into the output arm into at least two separate beams, means ("lame $\lambda$/" fig.4, QOP/M fig.5 et fig.6) of generating, in one of these two beams, an additional delay of $\lambda$/4 between the polarizations originating from the measurement arm and the reference arm of the interferometer, and means (MP/M1, MP/M2, MP/M3 fig.5 et fig.6) of reintroducing together the two beams thus processed into the Wollaston prism (W) such that, on output from the latter, there are then four light beams (A, B, C, D fig.4).

6. Device according to claim 5, such that the separator means comprise a single non-polarizing separator plate (BSP/M).

7. Device according to one of claims 5 or 6, such that the delaying means comprise a quarter-wave plate (QOP/M).

**8.** Device according to one of claims 5 to 7, such that the Wollaston prism (W) is arranged in a pupil plane.

**9.** Device according to one of claims 5 to 8, such that it also comprises means (DOP/M fig.5) to arbitrarily orient the polarizations of four incident beams relative to the Wollaston prism's (W) own axes.

**10.** Device according to claim 9, such that the means of orientation comprise a half-wave plate (DOP/M) preceding the Wollaston prism (W).

**11.** Method for measuring the contrast of fringes in a full-field polarized light Michelson interferometer comprising at least one reference arm and one measurement arm co-operating with an output arm so as to produce an optical coherence tomography system (OCT), the method comprising a deflection of two incoming perpendicular polarizations in two different emerging directions and for a simultaneous production of interferograms in different phase states, by means of a Wollaston prism (W) situated in said output arm.

**12.** Method according to claim 11, such that it comprises measurements for path differences differing by $\lambda/2$ or $\lambda/4$.

**13.** Method according to claim 12, such that it comprises at least two measurements, strictly simultaneous and in phase opposition.

**14.** Method according to one of claims 11 to 13, such that it comprises four measurements, a separation into two of a beam entering the output arm, a generation, in one of the two beams produced, of an additional delay of $\lambda/4$ between the polarizations originating from the measurement arm and the reference arm of the interferometer, and a reintroduction of the two beams thus processed into the Wollaston prism (W) such that, on output from the latter, there are then four light beams.

**15.** Method according to claim 14, such that it also comprises an arbitrary orientation of the polarizations of the four incident beams relative to the Wollaston prism's own axes.

**16.** Method according to claim 15, such that the measurements on the four beams are carried out simultaneously.

**17.** Method according to one of claims 1 to 16, such that it comprises, in the measurement arm, a compensation for the effects of focal chromatism of the eye.

**18.** Method according to one of claims 1 to 17, such that it comprises, in the reference arm, means for compensating for the dispersion of the path differences.

**19.** Method according to one of claims 1 to 18, such that it comprises a control of the wave front analyser (SH) obliging it to work in defocussed mode.

**20.** System for examining the eye by *in vivo* tomography, comprising:

   - a Michelson interferometer, comprising at least one measurement arm and one reference arm co-operating with an output arm in order to produce a full-field OCT setup,
   - adaptive optical means, arranged between the measurement arm of the interferometer and an eye to be examined or within said measurement arm, carrying out the correction of the wavefronts originating from the eye as well as those reaching the eye, and
   - means of detection, arranged downstream of the interferometer or within its output arm, making it possible to carry out the interferometric measurement according to the optical coherence tomography (OCT) principle, such that it also comprises a device according to any one of claims 1 to 10.

**21.** System for examining the eye according to claim 20, such that it also comprises a sighting device comprising at least one moving target having a programmable shape and trajectory, said target being displayed on an appropriate screen, visible by both eyes, during the examination period.

**22.** System according to one of claims 20 or 21, such that the reference source (SLD) is inserted into the optical path between the adaptive optical means (MD) and the eye to be examined (OEX).

**23.** System according to one of claims 20 to 22, such that it comprises, in the measurement arm, means for compensating for the effects of focal chromatism of the eye.

24. System according to one of claims 20 to 23, such that it comprises, in the reference arm, means for compensating for the dispersion of the path differences.

Lentille — Source
Polariseur —
Modulateur
Analyseur
Miroir de
Référence
Caméra
CCD
Objectifs
identiques
Lame λ/4 —
Cube
séparateur
polarisant
Objectif de
chambre
Echantillon

FIG.1

Prisme de Wollaston
Polarisation V
Polarisation H

FIG.2

Bras de sortie du Michelson

Référence = a1 → Mesure = a2

Axes du prisme de Wollaston

Objectif —
Prisme de Wollaston

Ah        Av

Détecteur

FIG.3

16

FIG.4

FIG.5

**FIG. 6**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

*   US 7079255 B